# EUROPEAN PATENT SPECIFICATION

(11) **EP 4 238 600 B1**
(45) Date of publication and mention of the grant of the patent: **12.02.2025**
(21) Application number: 21909143.6
(22) Date of filing: 08.12.2021
(51) Int. Cl.: A61M 16/00, G10K 11/16, F01N 1/10

(54) **VENTILATION SOUND-DIMINISHING APPARATUS AND VENTILATION THERAPY DEVICE**
VORRICHTUNG ZUR BEATMUNG ZUR KLANGMINDERUNG UND BEATMUNGSTHERAPIEVORRICHTUNG
APPAREIL DE RÉDUCTION DE SON DE VENTILATION ET DISPOSITIF DE THÉRAPIE DE VENTILATION

(30) Priority: 22.12.2020 CN 202011530641
(43) Date of publication of application: 06.09.2023
(73) Proprietor: BMC Medical Co., Ltd., Beijing 100142 (CN)
(72) Inventor: BI, Weidong, Beijing 100041 (CN); YI, Pinghu, Beijing 100041 (CN); ZHUANG, Zhi, Beijing 100041 (CN); ZHI, Jianxin, Beijing 100041 (CN)
(74) Representative: Grünecker Patent- und Rechtsanwälte PartG mbB
(86) International application number: PCT/CN2021/136280
(87) International publication number: WO 2022/135146

(56) References cited:
- WO-A1-2005/097244
- WO-A1-2020/121255
- WO-A2-2006/017398
- CN-A- 107 245 964
- CN-A- 108 729 367
- CN-A- 109 708 385
- CN-A- 112 020 743
- CN-A- 112 587 774
- CN-U- 202 418 373
- CN-U- 205 055 109
- CN-U- 215 024 279
- US-A1- 2006 060 418
- US-A1- 2018 340 328
- ANONYMOUS: "Sinowell 2018 USA Catalog", SINOWELL, 1 January 2018 (2018-01-01), XP055826145, Retrieved from the Internet <URL:https://www.sino-well.com/Upload/Catalog/201803301531246031.pdf> [retrieved on 20210720]

## Description

### FIELD

The present disclosure relates to the technical field of sound diminishing for ventilation therapy devices, in particular to a ventilation sound-diminishing apparatus and a ventilation therapy device.

### BACKGROUND

In ventilation therapy devices, such as ventilators, a turbomachine is usually used to deliver gas flow. As a power device and a main mechanical component for gas delivery, the turbomachine converts the energy in other forms into the mechanical energy for the rotation of an impeller, and the rotating impeller transfers the energy to the continuously flowing gas. Fans account for a great proportion in turbomachines, and centrifugal fans and axial fans are commonly used types of fans.

In using a ventilator, the motor of the fan produces certain noise, which will affect the patient. Therefore, it is necessary to treat the noise generated by the motor in the ventilator.

At present, a commonly used noise elimination means is a noise diminishing box, which is to say, a noise diminishing box having a soft wrapping material and a resistive sound diminishing material is provided inside the ventilator, and the noise diminishing box wraps the motor. However, the noise diminishing box is large in size, and occupies a large space in the ventilator, resulting in increased size of the ventilator. In addition, such a noise diminishing box can treat the highfrequency noise well, but has limited capability of low-frequency noise elimination. Moreover, the structural complexity of the noise diminishing box may easily result in excitation and superposition of the sound field in the box. All of those factors may have impacts on the comfort of the user.

US 2006/060418 A1 discloses a compact noise silencer for an air blower that forms the inlet and directs air into a burner for air heating for various applications, comprising an L-shaped back wall having a sound adsorbent media and side walls defining a triangular enclosure, an inlet opposite one portion of the back wall and a central outlet opposite the back wall having a filter directing air into a blower, wherein the sound adsorbent material includes a plurality of spaced projections to improve sound attenuation, which may be concave or convex.

WO 2006/017398 A2 discloses an apparatus for reducing the noise generated by compressors used in mechanical ventilators, comprising a noise-attenuating gas glow path having a plurality of chambers interconnected by flow tubes, wherein the dimensions of the chambers and the flow tubes are selected so that an impedance mismatch is created, and the perforated tube has a port through which gas is accepted and at least one exterior tube through which gas exits.CN 205055109 U discloses a gas noise reduction device comprising a casing with gas inlet and outlet, wherein the casing is divided into three sound attenuating chambers. The casing comprises a resistance wall which includes porous inner wall and an outer wall, wherein sound aborbing materials are sandwiched in between.

WO 2020/121255 A1 discloses an apparatus for providing air at positive pressure for respiratory therapy, comprising a flow tube array including a plurality of low tubes structured and arranged to extend from the base plate into the second inlet muffler chamber, so that the air flow path extends from second inlet muffler chamber through the flow tube array and into the third inlet muffler chamber.

WO 2005/097244 A1 discloses a ventilator comprising gas inlet with a flow tube array forming the inlet and arranged to allow laminar flow to provide a defined pressure drop.

Sinowell 2018 USA Catalog, XP055826145 discloses an inline duct silencer comprising sound reducing acoustic foam and is easy to connect to the air power equipment without affect airflow. CN 109708385 A discloses an air duct applicable to an air feeding system of a refrigerator, comprising an airflow channel, at least one side plate and a row of partition plates distributed along the air flow direction, so as to form a silencing cavity between any two adjacent partition plates in the row of partition plates; and the silencing cavities are used for eliminating or lowering airflow noise of airflow flowing through the airflow channel.

### SUMMARY

The invention is defined by claim 1 and its appended dependent claims.

An object of the present disclosure is to provide a ventilation sound-diminishing apparatus, which has a simple structure, and can effectively reduce the low-frequency noise in an intended elimination frequency band, thereby can improve the comfort of use of a ventilation therapy device when it is fitted in a ventilation therapy device.

To attain the above-mentioned object, the present disclosure provides a ventilation sound-diminishing apparatus, which comprises a sound-diminishing housing having a gas flow channel; the flow channel side walls of the gas flow channel comprise a first flow channel side wall and a second flow channel side wall arranged opposite to each other in a first direction; a plurality of first scattering bodies extending toward the interior of the gas flow channel and arranged in a plurality of rows and columns at intervals are provided on the inner surface of the first flow channel side wall; a plurality of second scattering bodies extending toward the interior of the gas flow channel and arranged in a plurality of rows and columns at intervals are provided on the inner surface of the second flow channel side wall; a preset distance is maintained between the first scattering bodies and the second scattering bodies in the first direction.

In the technical scheme, the flow channel side walls of the gas flow channel comprise a first flow channel side wall and a second flow channel side wall that are arranged opposite to each other in the first direction, a plurality of first scattering bodies extending toward the interior of the gas flow channel and arranged in a plurality of rows and a plurality of columns at intervals are provided on the inner surface of the first flow channel side wall to form a first array structure, a plurality of second scattering bodies extending toward the interior of the gas flow channel and arranged in a plurality of rows and a plurality of columns at intervals are provided on the inner surface of the second flow channel side wall to form a second array structure, and a preset distance is maintained between the first scattering bodies and the second scattering bodies in the first direction. Thus, the gas flow can flow through the gas flow channel, and, when the acoustic wave passes through the first array structure and the second array structure in the gas flow channel, a specific frequency of the acoustic wave in an interval (e.g., 400 Hz to 520 Hz, based on the wavelength of acoustic wave in the frequency band) will be attenuated owing to the oscillation in the array structure, thereby an effect of decreasing the intensity of the acoustic wave in the frequency interval is attained. Therefore, the ventilation sound-diminishing apparatus has a simple structure, can effectively reduce low-frequency noise in an intended elimination frequency band, thereby can improve the comfort of use of a ventilation therapy device when the ventilation sound-diminishing apparatus is fitted in the ventilation therapy device.

Furthermore, the first flow channel side wall is a first straight and flat flow channel side wall, the second flow channel side wall is a second straight and flat flow channel side wall, and the first straight and flat flow channel side wall and the second straight and flat flow channel side wall are arranged in parallel to each other.

Furthermore, the first scattering bodies and the corresponding second scattering bodies are aligned to each other in the first direction.

Furthermore, the length of the sound-diminishing housing in the extension direction of the gas flow channel is greater than the width of the cross section of the sound-diminishing housing, so that the gas flow channel is formed as an elongated flow channel; wherein the number of the scattering bodies in the cross-sectional direction of the gas flow channel is defined as a number of columns, the number of the scattering bodies arranged in the extension direction of the gas flow channel is defined as a number of rows, and the number of columns is smaller than the number of rows in the elongated flow channel.

Furthermore, the first scattering bodies and the second scattering bodies are solid ones; or the first scattering bodies and the second scattering bodies are hollow ones.

Furthermore, the first scattering bodies and the second scattering bodies are cylinders.

Furthermore, the first scattering bodies and the second scattering bodies are fins, which are in the same orientation and arranged perpendicular to the gas flow direction within the gas flow channel.

Furthermore, each of the fins comprises a cylinder and wings extending opposite to each other on the outer surface of the cylinder.

Furthermore, the first flow channel side wall and the second flow channel side wall are elastic side walls; and/or the outer surfaces of the first scattering bodies and the second scattering bodies are elastic outer surfaces.

In addition, the cross section of the sound-diminishing housing is rectangular or square.

Furthermore, the preset distance is adjustable.

Furthermore, the first scattering bodies and/or the second scattering bodies comprise telescopically fitted tubing subs, and the tubing subs telescope to adjust the preset distance;
and/or,
the flow channel side walls of the gas flow channel connected between the first flow channel side wall and the second flow channel side wall comprise telescopically fitted side wall stubs, and the side wall stubs telescope to adjust the preset distance.

Finally, the present disclosure provides a ventilation therapy device, which comprises any of the ventilation sound-diminishing apparatuses described above, wherein the gas flow channel serves as a channel section of a gas delivery channel of the ventilation therapy device. Thus, as described above, the noise of the ventilation therapy device is significantly reduced, and the overall quality of the ventilation therapy device is improved. The ventilation therapy device may be a ventilator, a high-flow humidifying oxygen therapy device, or the like.

Other features and advantages of the present disclosure will be further detailed in the following embodiments.

### BRIEF DESCRITION OF THE DRAWINGS

The accompanying drawings are provided herein to facilitate further understanding on the present disclosure and constitute a part of this document. They are used in conjunction with the following embodiments to explain the present disclosure, but are not intended to constitute any limitation to the present disclosure. In the figures:
Fig. 1 is an isometric structural view of the ventilation sound-diminishing apparatus provided in an embodiment of the present disclosure from a viewing angle;
Fig. 2 is an isometric structural view of the ventilation sound-diminishing apparatus in Fig. 1 from another viewing angle;
Fig. 3 is an end view of the ventilation sound-diminishing apparatus in Fig. 1;
Fig. 4 is a sectional view of the ventilation sound-diminishing apparatus in Fig. 1 along the gas flow channel;
Fig. 5 is a schematic structural diagram of the fins in another ventilation sound-diminishing apparatus provided in an embodiment of the present disclosure, in which the first scattering bodies and the second scattering bodies are fins;
Fig. 6 is a schematic structural diagram of the ventilation sound-diminishing apparatus in Fig. 1 mounted on the gas outlet of a fan;
Fig. 7 is a curve chart of elimination of the low-frequency noise at an intended elimination frequency with the ventilation sound-diminishing apparatus provided in an embodiment of the present disclosure.

### Reference Numbers

1 - gas flow channel, 2 - sound-diminishing housing, 3 - first straight and flat flow channel side wall, 4 - second straight and flat flow channel side wall, 5 - first scattering body, 6 - second scattering body, 7 - cylinder, 8 - wing, 9 - ventilation sound-diminishing apparatus, 10 - fan.

### DETAILED DESCRPTION

Some embodiments of the present disclosure will be detailed below with reference to the accompanying drawings. It should be understood that the embodiments described herein are only provided to describe and explain the present disclosure, but are not intended to constitute any limitation to the present disclosure.

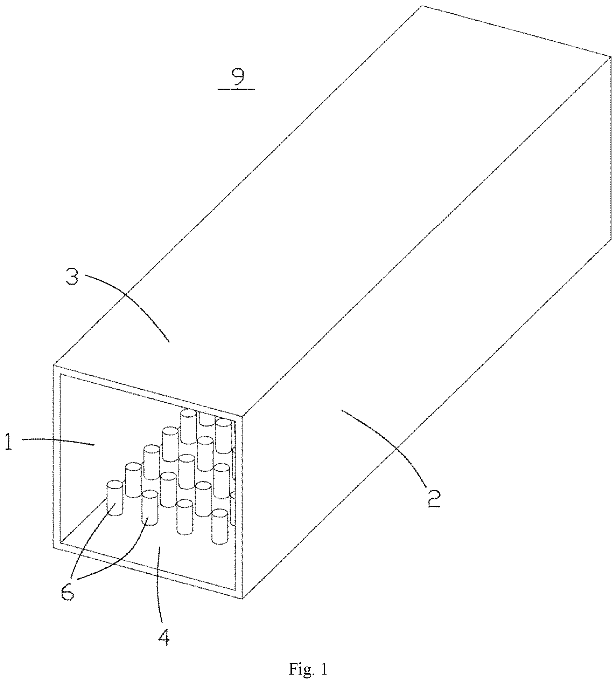

As shown in Figs. 1 - 4, the ventilation sound-diminishing apparatus 9 provided by the present disclosure comprises a sound-diminishing housing 2 having a gas flow channel 1; the flow channel side walls of the gas flow channel 1 comprise a first flow channel side wall and a second flow channel side wall arranged opposite to each other in a first direction, wherein, a plurality of first scattering bodies 5 extending toward the interior of the gas flow channel 1 and arranged in a plurality of rows and columns at intervals are provided on the inner surface of the first flow channel side wall to form a first array structure; a plurality of second scattering bodies 6 extending toward the interior of the gas flow channel 1 and arranged in a plurality of rows and columns at intervals are provided on the inner surface of the second flow channel side wall to form a second array structure; and a preset distance is maintained between the first scattering bodies 5 and the second scattering bodies 6 in the first direction, which is to say, a preset distance is maintained between the first array structure and the second array structure.

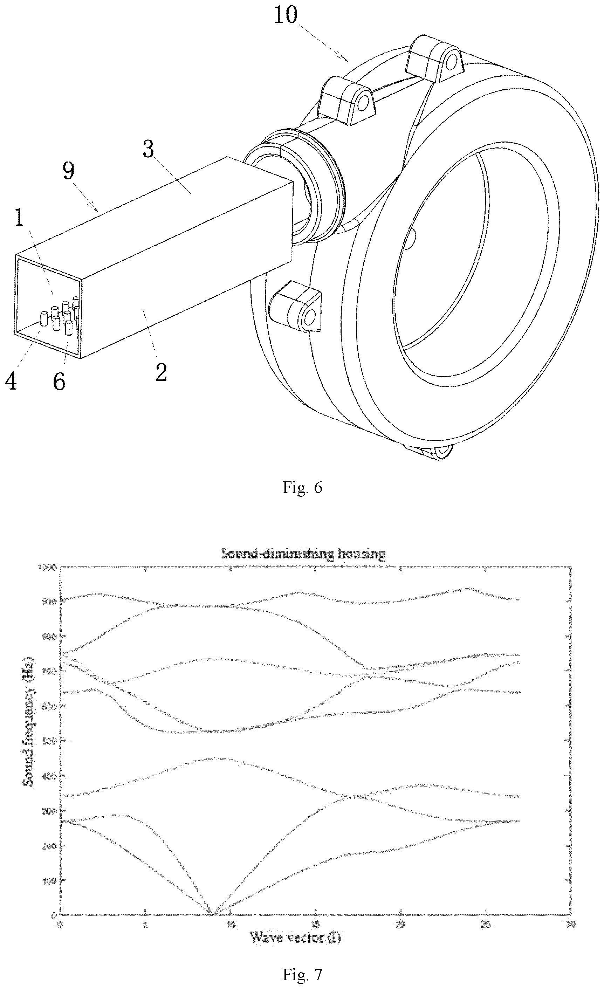

The flow channel side walls of the gas flow channel 1 comprise a first flow channel side wall and a second flow channel side wall that are arranged opposite to each other in the first direction, a plurality of first scattering bodies 5 extending toward the interior of the gas flow channel and arranged in a plurality of rows and a plurality of columns at intervals are provided on the inner surface of the first flow channel side wall to form a first array structure, a plurality of second scattering bodies 6 extending toward the interior of the gas flow channel and arranged in a plurality of rows and a plurality of columns at intervals are provided on the inner surface of the second flow channel side wall to form a second array structure, and a preset distance is maintained between the first scattering bodies 5 and the second scattering bodies 6 in the first direction. Thus, the gas flow can flow through the gas flow channel 1, and, when the acoustic wave passes through the first array structure and the second array structure in the gas flow channel, a specific frequency of the acoustic wave in an interval, e.g., the wavelength of the acoustic wave in an interval of 400 Hz to 520 Hz as shown in Fig. 7, is attenuated owing to the oscillation in the array structure, thereby an effect of decreasing the intensity of the frequency in the interval is attained. Therefore, the ventilation sound-diminishing apparatus has a simple structure, and can effectively reduce the low-frequency noise in an intended elimination frequency band, thereby can improve the comfort of use of a ventilation therapy device when it is fitted in a ventilation therapy device.

In addition, in the ventilation sound-diminishing apparatus, the first flow channel side wall and the second flow channel side wall may be in a variety of shapes; for example, the first flow channel side wall and the second flow channel side wall may be arc-shaped flow channel side walls or straight and flat flow channel side walls. Moreover, the first flow channel side wall and the second flow channel side wall may be arranged away from each other at one end and near each other at the other end; thus, the gas flow channel 1 is formed as a flow channel that is reduced gradually or expanded gradually in the gas flow direction.

For example, in an embodiment, as shown in Fig. 1, the first flow channel side wall is a first straight and flat flow channel side wall 3, the second flow channel side wall is a second straight and flat flow channel side wall 4, and the first straight and flat flow channel side wall 3 and the second straight and flat flow channel side wall 4 are arranged in parallel to each other, so that the gas can flow smoothly along the first straight and flat flow channel side wall 3 and the second straight and flat flow channel side wall 4 to pass through the first array structure and the second array structure smoothly, thereby the noise of the gas can be further reduced.

The first scattering bodies 5 and the second scattering bodies 6 may be formed on the respective flow channel side walls, e.g., straight and flat flow channel side walls, by insertion, laser welding, threaded connection, or integrally forming.

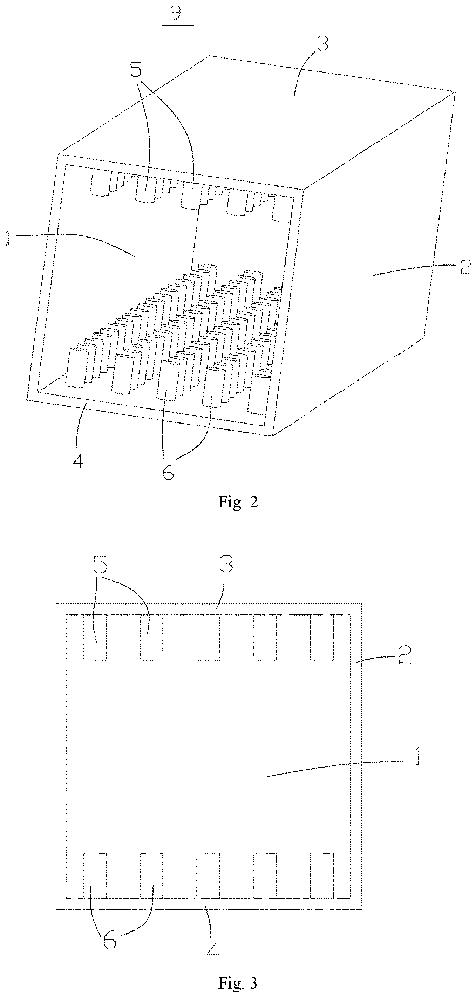

In addition, in an embodiment of the ventilation sound-diminishing apparatus, the first scattering bodies 5 arranged in a plurality of rows and a plurality of columns in the first array structure and the second scattering bodies 6 arranged in a plurality of rows and a plurality of columns in the second array structure may be arranged in a staggered manner. Alternatively, in another embodiment, as shown in Fig. 3, the first scattering bodies 5 are aligned to the second scattering bodies 6 in the first direction, thereby the effect of eliminating the low-frequency noise in the intended elimination frequency band can be further improved.

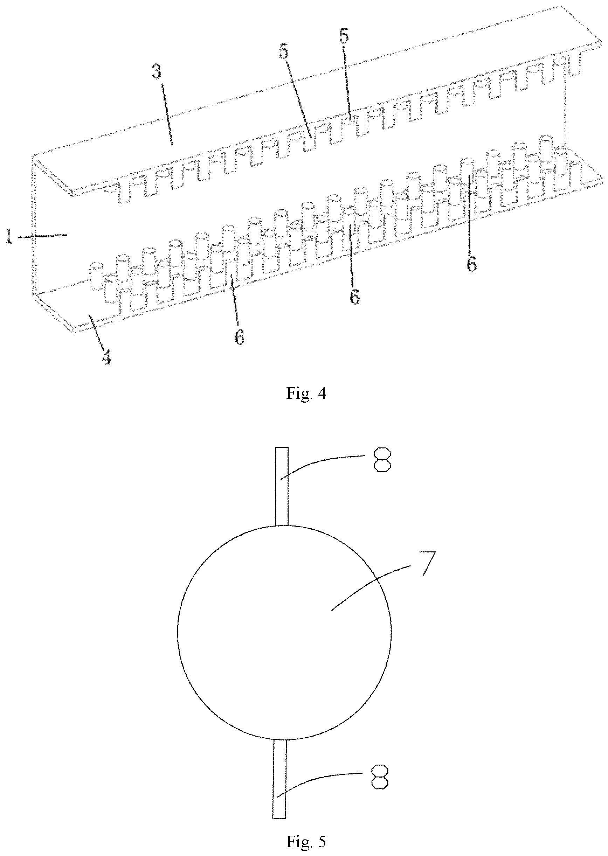

Moreover, the number of rows and number of columns in the first array structure may be selected according to the actual requirement, and the number of rows and number of columns in the second array structure may be selected according to the actual requirement; for example, the sound-diminishing housing 2 may be an elongated housing, which is to say, the length of the sound-diminishing housing 2 in the extension direction of the gas flow channel 1 is greater than the cross-sectional width of the sound-diminishing housing 2, so that the gas flow channel 1 is formed as an elongated flow channel; the number of the scattering bodies in the cross-sectional direction of the gas flow channel 1 is defined as number of columns, and the number of the scattering bodies in the extension direction of the gas flow channel 1 is defined as number of rows; in the elongated flow channel, the number of columns of the first scattering bodies 5 and of the second scattering bodies 6 is smaller than the number of rows, thereby the path of the acoustic wave can be lengthened to improve the noise elimination effect. For example, preferably the number of columns may be 4 - 10, further preferably is 5; preferably the number of rows is 15 - 20, further preferably is 18. For example, in the embodiments shown in Figs. 1 and 4, in the first array structure and the second array structure, the number of columns of the first scattering bodies 5 and of the second scattering bodies 6 is 5, and the number of rows is 18.

In addition, in the ventilation sound-diminishing apparatus, the first scattering bodies 5 and the second scattering bodies 6 may be solid ones; for example, they may be solid lead bodies; thus, a material with required density in the energy band calculation may be selected according to the distribution of the energy band of the acoustic wave to support the solid bodies.

In addition, the first scattering bodies 5 and the second scattering bodies 6 may be hollow ones, which is to say, cavities are formed in the scattering bodies. The cavities may be in a variety of shapes, preferably consistent with the outer contours of the scattering bodies. For example, if the scattering bodies are cylindrical, the cavities of the cylinders may be cylindrical.

Moreover, the first scattering bodies 5 and the second scattering bodies 6 may have a variety of outer contour shapes, which may be selected according to the actual requirement. For example, in an embodiment, the first scattering bodies 5 and the second scattering bodies 6 may be spheres; alternatively, in another embodiment, as shown in Figs. 1, 2 and 4, the first scattering bodies 5 and the second scattering bodies 6 are cylinders. Thus, the outer circumferential surfaces of the cylinders facilitate gas flow to pass through, and can effectively eliminate the low-frequency noise in the intended elimination frequency band. Furthermore, the dimensions of the scattering bodies, for example, the diameters of the cylinders, may be selected according to the actual requirements. For example, the diameters of the scattering bodies may be 3 - 8 mm, further preferably are 4 mm.

In addition, in other embodiments, the first scattering bodies 5 and the second scattering bodies 6 are fins, which are in the same orientation and arranged perpendicular to the gas flow direction in the gas flow channel 1. Thus, gas flow guiding spacing can be formed between the fins, to facilitate the gas flow to pass through the gas flow channel; besides, the surfaces of the fins can attain an effect of effectively eliminating the low-frequency noise in the intended elimination frequency band.

Moreover, the fins may be straight and flat plates or wavy plates; alternatively, in other embodiments, as shown in Fig. 5, each of the fins comprises a cylinder 7 and wings 8 protruding opposite to each other from the outer surface of the cylinder respectively. Thus, the fins having such a structure have the advantages of a cylinder and a plate, and can attain a more effective effect of eliminating of the low-frequency noise in the intended elimination frequency band while facilitating the gas flow to pass through the gas flow channel 1.

Moreover, at least one of the first flow channel side wall and the second flow channel side wall, e.g., the first straight and flat flow channel side wall 3 and the straight and flat flow channel side wall 4, may be elastic side walls; for example, the first straight and flat flow channel side wall 3 and the second straight and flat flow channel side wall 4 are elastic side walls, such as silicone rubber side walls, which has elasticity and rigidity, so as to attain an effect of eliminating elastic waves while supporting the mounted scattering bodies.

In addition, the outer surfaces of the first scattering bodies 5 and the second scattering bodies 6 are elastic outer surfaces, for example, elastic sheaths are sheathed on the outer surfaces of the scattering bodies. Thus, the elastic outer surfaces also attain an effect of eliminating elastic waves.

Moreover, in the first direction, the thickness of the first flow channel side wall and the second flow channel side wall, such as the straight and flat flow channel side walls, and the height of the scattering bodies, may be selected according to the actual requirement. For example, in an embodiment, preferably, the thickness of the straight and flat flow channel side walls may be 2 - 5 mm, further preferably is 2 mm; preferably the height of the scattering bodies may be 10 - 15 mm, further preferably is 10 mm.

Moreover, the flow channel side walls (e.g., the left and right vertical flow channel side walls in Fig. 3) between the first flow channel side wall and the second flow channel side wall, e.g., the first straight and flat flow channel side wall 3 and the second straight and flat flow channel side wall 4 may be straight side walls or arc-shaped side walls. For example, in an embodiment, the flow channel side walls between the first flow channel side wall and the second flow channel side wall, e.g., the first straight and flat flow channel side wall 3 and the second straight and flat flow channel side wall 4, are straight side walls, so that the cross section of the sound-diminishing housing 2 is rectangular or square, thereby attaining a better noise elimination effect while facilitating the gas flow to pass through.

In addition, in an optional embodiment of the ventilation sound-diminishing apparatus, the preset distance between the first heating body 5 and the second scattering body 6 is adjustable. Thus, the preset distance may be adjusted according to the low-frequency noise in the intended elimination frequency band, thereby the ventilation sound-diminishing apparatus can eliminate a greater range of noise, and is applicable to the noise reduction and elimination in different occasions.

Of course, the adjustment of the preset distance may be realized by means of different structures. For example, in an embodiment, at least one of the first scattering body 5 and the second scattering body 6 comprises a telescopically fitted tubing sub. Thus, the tubing subs serve as the hollow bodies, and telescope to adjust the preset distance. Therefore, the noise can be eliminated more effectively.

In another embodiment, the two flow channel side walls of the gas flow channel 1 connected between the first flow channel side wall and the second flow channel side wall, e.g., the first straight and flat flow channel side wall 3 and the second straight and flat flow channel side wall 4 comprise a telescopically fitted side wall stubs respectively, and the side wall stubs telescope to adjust the preset distance, thereby the ventilation sound-diminishing apparatus can eliminate noise in a wider range, and is applicable to the noise reduction and elimination in different occasions.

In other embodiments, at least one of the first scattering body 5 and the second scattering body 6 comprises a telescopically fitted tubing subs; thus, the tubing subs serve as the hollow bodies, and telescope to adjust the preset distance; the two flow channel side walls of the gas flow channel 1 connected between the first flow channel side wall and the second flow channel side wall, e.g., the first straight and flat flow channel side wall 3 and the second straight and flat flow channel side wall 4, comprise a telescopically fitted side wall stubs respectively, and the side wall stubs telescope to adjust the preset distance.

Finally, as shown in Fig. 6, the present disclosure provides a ventilation therapy device, which comprises any of the ventilation sound-diminishing apparatuses 9 described above, wherein the gas flow channel 1 serves as a channel section of a gas delivery channel of the ventilation therapy device. For example, in Fig. 6, the ventilation sound-diminishing apparatus is arranged at the gas outlet of the fan 10. Of course, alternatively the ventilation sound-diminishing apparatus may be arranged at the gas inlet of the fan 10, or arranged at the gas inlet of the noise diminishing box of the ventilation therapy device.

Typically, the ventilation therapy device may be a ventilator, a high-flow humidifying oxygen therapy device, or the like. With the above ventilation sound-diminishing apparatus, these devices have significantly lowered noise during use, thereby the user's experience and the quality of the product are improved remarkably.

## Claims

1. A ventilation therapy device comprising a ventilation sound-diminishing apparatus, comprising a sound-diminishing housing (2) having a gas flow channel (1),
wherein the gas flow channel (1) serves as a channel section of a gas delivery channel of the ventilation therapy device,
wherein flow channel side walls of the gas flow channel (1) comprise a first flow channel side wall and a second flow channel side wall that are arranged opposite to and spaced apart from each other in a first direction,
**characterized in that**
a plurality of first scattering bodies (5) extending toward the interior of the gas flow channel (1) and arranged in a plurality of rows and a plurality of columns at intervals are provided on an inner surface of the first flow channel side wall, a plurality of second scattering bodies (6) extending toward the interior of the gas flow channel (1) and arranged in a plurality of rows and a plurality of columns at intervals are provided on an inner surface of the second flow channel side wall, and a preset distance is maintained between the first scattering bodies (5) and the second scattering bodies (6) in the first direction.

2. The ventilation therapy device of claim 1, wherein the first flow channel side wall is a first straight and flat flow channel side wall (3), the second flow channel side wall is a second straight and flat flow channel side wall (4), and the first straight and flat flow channel side wall (3) and the second straight and flat flow channel side wall (4) are arranged in parallel to each other.

3. The ventilation therapy device of claim 1 or 2, wherein the first scattering bodies (5) and the corresponding second scattering bodies (6) are aligned to each other in the first direction.

4. The ventilation therapy device of any of the preceding claims, wherein the length of the sound-diminishing housing (2) in the extension direction of the gas flow channel (1) is greater than the width of the cross section of the sound-diminishing housing (2), so that the gas flow channel (1) is formed as an elongated flow channel;
wherein the number of the scattering bodies in the cross-sectional direction of the gas flow channel (1) is defined as a number of columns, the number of the scattering bodies arranged in the extension direction of the gas flow channel (1) is defined as a number of rows, and the number of columns is smaller than the number of rows in the elongated flow channel.

5. The ventilation therapy device of any of the preceding claims, wherein the first scattering bodies (5) and the second scattering bodies (6) are solid ones; or the first scattering bodies (5) and the second scattering bodies (6) are hollow ones.

6. The ventilation therapy device of any of the preceding claims, wherein the first scattering bodies (5) and the second scattering bodies (6) are cylinders.

7. The ventilation therapy device of any of claims 1 - 5, wherein the first scattering bodies (5) and the second scattering bodies (6) are fins, which are in the same orientation and arranged perpendicular to the gas flow direction within the gas flow channel (1).

8. The ventilation therapy device of claim 7, wherein each of the fins comprises a cylinder (7) and wings (8) extending opposite to each other on the outer surface of the cylinder (7).

9. The ventilation therapy device of any of the preceding claims, wherein the first flow channel side wall and the second flow channel side wall are elastic side walls;
and/or,
the outer surfaces of the first scattering bodies (5) and the second scattering bodies (6) are elastic outer surfaces.

10. The ventilation therapy device of any of the preceding claims, wherein the cross section of the sound-diminishing housing (2) is rectangular or square.

11. The ventilation therapy device of any of the preceding claims, wherein the preset distance is adjustable.

12. The ventilation therapy device of claim 11, wherein the first scattering bodies (5) and/or the second scattering bodies (6) comprise telescopically fitted tubing subs, and the tubing subs telescope to adjust the preset distance;
and/or,
the flow channel side walls of the gas flow channel (1) connected between the first flow channel side wall and the second flow channel side wall comprise telescopically fitted side wall stubs, and the side wall stubs telescope to adjust the preset distance.

## Patentansprüche

1. Beatmungsbehandlungs-Vorrichtung, die eine Einrichtung zum Mindern von Beatmungsgeräusch umfasst, die ein Geräuschminderungs-Gehäuse (2) mit einem Gasstrom-Kanal (1) umfasst,
wobei der Gasstrom-Kanal (1) als ein Kanal-Teilabschnitt eines Gaszufuhr-Kanals der Beatmungsbehandlungs-Vorrichtung dient,
wobei Stromkanal-Seitenwände des Gasstrom-Kanals (1) eine erste Stromkanal-Seitenwand sowie eine zweite Stromkanal-Seitenwand umfassen, die einander in einer ersten Richtung gegenüberliegend und voneinander getrennt angeordnet sind,
**dadurch gekennzeichnet, dass**
eine Vielzahl erster Verteilungs-Körper (5), die sich auf den Innenraum des Gasstrom-Kanals (1) zu erstrecken und in einer Vielzahl von Reihen sowie einer Vielzahl von Spalten in Abständen angeordnet sind, an einer Innenfläche der ersten Stromkanal-Seitenwand vorhanden sind, eine Vielzahl zweiter Verteilungs-Körper (6), die sich auf dem Innenraum des Gasstrom-Kanals (1) zu erstrecken und in einer Vielzahl von Reihen sowie einer Vielzahl von Spalten in Abständen angeordnet sind, an einer Innenfläche der zweiten Stromkanal-Seitenwand vorhanden sind und ein vorgegebener Abstand zwischen den ersten Verteilungs-Körpern (5) und den zweiten Verteilungs-Körpern (6) in der ersten Richtung gehalten wird.

2. Beatmungsbehandlungs-Vorrichtung nach Anspruch 1, wobei die erste Stromkanal-Seitenwand eine erste gerade und flache Stromkanal-Seitenwand (3) ist, die zweite Stromkanal-Seitenwand eine zweite gerade und flache Stromkanal-Seitenwand (4) ist und die erste gerade und flache Stromkanal-Seitenwand (3) und die zweite gerade und flache Stromkanal-Seitenwand (4) parallel zueinander angeordnet sind.

3. Beatmungsbehandlungs-Vorrichtung nach Anspruch 1 oder 2, wobei die ersten Verteilungs-Körper (5) und die entsprechenden zweiten Verteilungs-Körper (6) in der ersten Richtung aufeinander ausgerichtet sind.

4. Beatmungsbehandlungs-Vorrichtung nach einem der vorangehenden Ansprüche, wobei die Länge des Geräuschminderungs-Gehäuses (2) in der Verlaufsrichtung des Gasstrom-Kanals (1) größer ist als die Breite des Querschnitts des Geräuschminderungs-Gehäuses (2), so dass der Gasstrom-Kanal (1) als ein länglicher Stromkanal ausgebildet ist;
wobei die Anzahl der Verteilungs-Körper in der Querschnittsrichtung des Gasstrom-Kanals (1) als eine Anzahl von Spalten definiert ist, die Anzahl der Verteilungs-Körper, die in der Verlaufsrichtung des Gasstrom-Kanals (1) angeordnet sind, als eine Anzahl von Reihen definiert ist und die Anzahl von Spalten kleiner ist als die Anzahl von Reihen in dem länglichen Stromkanal.

5. Beatmungsbehandlungs-Vorrichtung nach einem der vorangehenden Ansprüche, wobei die ersten Verteilungs-Körper (5) und die zweiten Verteilungs-Körper (6) massive Körper sind oder die ersten Verteilungs-Körper (5) und die zweiten Verteilungs-Körper (6) hohle Körper sind.

6. Beatmungsbehandlungs-Vorrichtung nach einem der vorangehenden Ansprüche, wobei die ersten Verteilungs-Körper (5) und die zweiten Verteilungs-Körper (6) Zylinder sind.

7. Beatmungsbehandlungs-Vorrichtung nach einem der Ansprüche 1 - 5, wobei die ersten Verteilungs-Körper (5) und die zweiten Verteilungs-Körper (6) Rippen sind, die die gleiche Ausrichtung haben und senkrecht zu der Gasstrom-Richtung in dem Gasstrom-Kanal (1) angeordnet sind.

8. Beatmungsbehandlungs-Vorrichtung nach Anspruch 7, wobei jede der Rippen einen Zylinder (7) und Flügel (8) umfasst, die sich entgegengesetzt zueinander an der Außenfläche des Zylinders (7) erstrecken.

9. Beatmungsbehandlungs-Vorrichtung nach einem der vorangehenden Ansprüche, wobei die erste Stromkanal-Seitenwand und die zweite Stromkanal-Seitenwand elastische Seitenwände sind;
und/oder
die Außenflächen der ersten Verteilungs-Körper (5) und der zweiten Verteilungs-Körper (6) elastische Außenflächen sind.

10. Beatmungsbehandlungs-Vorrichtung nach einem der vorangehenden Ansprüche, wobei der Querschnitt des Geräuschminderungs-Gehäuses (2) rechteckig oder quadratisch ist.

11. Beatmungsbehandlungs-Vorrichtung nach einem der vorangehenden Ansprüche, wobei der vorgegebene Abstand verstellt werden kann.

12. Beatmungsbehandlungs-Vorrichtung nach Anspruch 11, wobei die ersten Verteilungs-Körper (5) und/oder die zweiten Verteilungs-Körper (6) teleskopartig ineinandergepasste Röhren-Teilstücke umfassen und sich die Röhren-Teilstücke ineinanderschieben lassen, um den vorgegebenen Abstand zu verstellen;
und/oder
die Stromkanal-Seitenwände des Gasstrom-Kanals (1), die zwischen die erste Stromkanal-Seitenwand und die zweite Stromkanal-Seitenwand eingesetzt sind, teleskopartig ineinandergepasste Seitenwandabschnitte umfassen und sich die Seitenwandabschnitte ineinanderschieben lassen, um den vorgegebenen Abstand zu verstellen.

## Revendications

1. Dispositif de thérapie par ventilation comprenant un appareil de diminution du son de ventilation,
comprenant un logement de diminution de son (2) ayant un canal d'écoulement de gaz (1),
dans lequel le canal d'écoulement de gaz (1) sert de section de canal d'un canal de distribution de gaz du dispositif de thérapie par ventilation,
dans lequel les parois latérales de canal d'écoulement du canal d'écoulement de gaz (1) comprennent une première paroi latérale de canal d'écoulement et une seconde paroi latérale de canal d'écoulement qui sont agencées opposées l'une à l'autre, et espacées l'une de l'autre, dans un premier sens,
**caractérisé en ce que**
une pluralité de premiers corps de diffusion (5) s'étendant vers l'intérieur du canal d'écoulement de gaz (1) et agencés dans une pluralité de rangées et une pluralité de colonnes par intervalles sont prévus sur une surface interne de la première paroi latérale de canal d'écoulement, une pluralité de seconds corps de diffusion (6) s'étendant vers l'intérieur du canal d'écoulement de gaz (1) et agencés selon une pluralité de rangées et une pluralité de colonnes par intervalles sont prévus sur une surface interne de la seconde paroi latérale de canal d'écoulement, et une distance prédéfinie est maintenue entre les premiers corps de diffusion (5) et les seconds corps de diffusion (6) dans le premier sens.

2. Dispositif de thérapie par ventilation selon la revendication 1, dans lequel la première paroi latérale de canal d'écoulement est une première paroi latérale de canal d'écoulement droite et plate (3), la seconde paroi latérale de canal d'écoulement est une seconde paroi latérale de canal d'écoulement droite et plate (4), et la première paroi latérale de canal d'écoulement droite et plate (3) et la seconde paroi latérale de canal d'écoulement droite et plate (4) sont agencées en parallèle l'une par rapport à l'autre.

3. Dispositif de thérapie par ventilation selon la revendication 1 ou 2, dans lequel les premiers corps de diffusion (5) et les seconds corps de diffusion (6) correspondants sont alignés l'un par rapport à l'autre dans le premier sens.

4. Dispositif de thérapie par ventilation selon l'une quelconque des revendications précédentes, dans lequel la longueur du logement de diminution de son (2) dans le sens de l'extension du canal d'écoulement de gaz (1) est supérieure à la largeur de la section transversale du logement de diminution de son (2), de sorte que le canal d'écoulement de gaz (1) est formé sous la forme d'un canal d'écoulement allongé ;
dans lequel le nombre de corps de diffusion dans le sens transversal du canal d'écoulement de gaz (1) est défini comme un nombre de colonnes, le nombre de corps de diffusion agencés dans le sens de l'extension du canal d'écoulement de gaz (1) est défini comme un nombre de rangées, et le nombre de colonnes est inférieur au nombre de rangées dans le canal d'écoulement allongé.

5. Dispositif de thérapie par ventilation selon l'une quelconque des revendications précédentes, dans lequel les premiers corps de diffusion (5) et les seconds corps de diffusion (6) sont pleins ; ou les premiers corps de diffusion (5) et les seconds corps de diffusion (6) sont creux.

6. Dispositif de thérapie par ventilation selon l'une quelconque des revendications précédentes, dans lequel les premiers corps de diffusion (5) et les seconds corps de diffusion (6) sont des cylindres.

7. Dispositif de thérapie par ventilation selon l'une quelconque des revendications 1 à 5, dans lequel les premiers corps de diffusion (5) et les seconds corps de diffusion (6) sont des ailettes, qui se trouvent dans la même orientation et sont agencés perpendiculaires au sens d'écoulement de gaz à l'intérieur du canal d'écoulement de gaz (1).

8. Dispositif de thérapie par ventilation selon la revendication 7, dans lequel chacune des ailettes comprend un cylindre (7) et des ailes (8) s'étendant opposées l'une par rapport à l'autre sur la surface externe du cylindre (7).

9. Dispositif de thérapie par ventilation selon l'une quelconque des revendications précédentes, dans lequel la première paroi latérale de canal d'écoulement et la seconde paroi latérale de canal d'écoulement sont des parois latérales élastiques ; et/ou,
les surfaces externes des premiers corps de diffusion (5) et des seconds corps de diffusion (6) sont des surfaces externes élastiques.

10. Dispositif de thérapie par ventilation selon l'une quelconque des revendications précédentes, dans lequel la section transversale du logement de diminution de son (2) est rectangulaire ou carrée.

11. Dispositif de thérapie par ventilation selon l'une quelconque des revendications précédentes, dans lequel la distance prédéfinie est ajustable.

12. Dispositif de thérapie par ventilation selon la revendication 11, dans lequel les premiers corps de diffusion (5) et/ou les seconds corps de diffusion (6) comprennent des essieux de tubulure télescopiquement ajustés, et le télescope à essieux de tubulure pour ajuster la distance prédéfinie ; et/ou,
les parois latérales de canal d'écoulement du canal d'écoulement de gaz (1) raccordées entre la première paroi latérale de canal d'écoulement et la seconde paroi latérale de canal d'écoulement comprennent des essieux de paroi latérale télescopiquement ajustés, et le télescope à essieux de paroi latérale pour ajuster la distance prédéfinie.
